# EUROPEAN PATENT APPLICATION

(11) **EP 1 072 241 A1**
(43) Date of publication of application: **31.01.2001**
(21) Application number: 00902944.8
(22) Date of filing: 14.02.2000
(51) Int. Cl.: A61F 5/02

(54) **JOINT FITTING**

(30) Priority: 15.02.1999 JP 3568099
(71) Applicant: TEIJIN LIMITED, Osaka-shi Osaka 541-0054 (JP)
(72) Inventor: TAKAICHI, Satoshi, Teijin Limited, Tokyo 191-0065 (JP)
(74) Representative: Marsh, Roy David
(86) International application number: JP0000789
(87) International publication number: WO0047142

(57) **Abstract**

The present invention relates to a joint brace comprising a joint angle fixation portion to fix a joint of a limb while the joint is bent at a right angle, by fixing the joint at the upper and/or lower limbs of the joint, and a treatment device installing portion to enable treatment with a treatment device, and also the treatment device installing portion being so configured as to allow the treatment device to be directed towards the cavity of the joint for treatment.

## Description

### Field of the Invention

The present invention relates to a joint brace with which a patient with a disorder in a joint can safely have a treatment device attached to an elbow or knee joint for treatment or diagnosis. More specifically, the present invention relates to a joint brace for fixing an elbow or knee joint, which helps a device for treatment or diagnosis to be firmly attached to the affected joint, thereby insuring the therapeutic effect.

### Background Art

A wide variety of diseases including osteoarthritis and osteochondritis dissecans affect the elbow or knee joint, but even today there is no established therapeutic method for treating those articular diseases. Therapies based on the use of a device emitting ultrasonic waves, magnetic fluxes, or electromagnetic waves have been tried for the treatment of those diseases.

The therapy based on the use of such a device generally continues long often lasting from several days to several months. To insure the therapeutic effect of the device, it is necessary for the patient to keep the device at the same position and angle with respect to the affected joint for several minutes to several hours for a day's treatment. Sometimes, the patient is required to be reposed or to be applied to a joint flexed at a right angle when the device is applied to a joint.

An example of such a joint brace is disclosed in Japanese Patent Laid-Open No. TOKKAIHEI 7-255762 which describes a brace for fixing the arthritic knee joint. This brace has a hinge part consisting of an elastic body with a springy property, and upper and lower legs fixed to lessen the load imposed on the knee joints. However, this device is not designed on the premise that the patient might receive treatment on the affected knee joint while wearing the brace. Therefore, if a device such as a head to emit ultrasonic waves were attached to the brace for ultrasonic therapy, the device could not be kept at a proper position during treatment, because fixation only with the upper and lower legs of the brace would allow the knee joint to move freely. Moreover, if the brace were attached and removed each time treatment is introduced and discontinued, it would not be always possible to insure the brace to be properly fixed at the same position. Because of this, such a brace will be inadequate to be applied to a joint requiring attachment of a therapeutic device.

Conventionally, to apply the radiation part of a ultrasonic treatment device onto the affected elbow or knee joint, the patient rests his affected elbow or knee joint on the floor of an examination bed, and a surgeon or the patient himself holds the radiation part against the affected joint by hand to apply ultrasonic waves to that joint. This therapeutic method, however, is not only time consuming and burdensome to the patient, but makes it difficult to apply the device against the same site particularly when the therapy continues as long as several months. Therefore, it often occurs that the therapeutic device shifts from the proper position during the treatment period, its therapeutic effect is impaired therewith, and, it has been suggested, in the worst cases its therapeutic effect may be completely aborted.

Another method which is often adopted at site in hospitals consists of fixing a treatment device onto the affected elbow or knee joint with belts wound around it. However, such a method can hardly be applied for fixing a device to an elbow or knee joint flexed at a right angle, a posture often taken by the patient when he reposes the joint, and even if forceful application thereof were successfully managed, it might give a too much pressure onto the lesion, causing it to aggravate. Moreover, with this method, repeated application of the device at the same proper position would be difficult as in the above example where positioning of the device is achieved manually.

### Disclosure of the Invention

This invention provides a joint brace which, even when applied together with a therapeutic device to an affected joint, can fix the therapeutic device so stably that the device will not change its position and direction with respect to the lesion no matter how repeatedly the therapeutic device is attached and removed to and from the affected joint, will not give a too much pressure on the affected elbow or knee joint during application, and will allow the joint even flexed at a right angle, that is, a posture often taken by the patient when he reposes the joint, to keep that position.

To achieve the above object, the present inventors have studied strenuously and found that a brace described below will meet the object.

The present invention will provide a joint brace comprising a joint angle fixation portion to fix a joint of a limb while the joint is bent at a right angle, by being fixed at the upper limb and/or lower one encompassing, and comprising a treatment device installing portion to facilitate the therapy based on the use of that device, and characterized in that the treatment device installing portion is so configured as to allow the device to apply treatment towards the capsule of the joint.

The present invention will further provide a joint brace of which the joint angle fixation portion comprises a distal fixation part to be attached to the lower limb of a joint, and an L-shaped fixation section extending from the distal fixation part along the lateral aspect of the lower limb until it contacts with the dorsal aspect of the upper limb close to the joint.

This invention will still further provide a joint brace of which the joint angle fixation portion comprises a distal fixation part to be attached to the lower limb of a joint for fixing the joint brace, an L-shaped fixation section extending from the distal fixation part along the lateral aspect of the lower limb until it contacts with the dorsal aspect of the upper limb close to the joint, and a treatment device installing portion is attached from an L-shaped end of the L-shaped fixation section and arranged in parallel with and opposite to the L-shaped fixation section. Particularly, this invention will provide a joint brace of which the treatment device installing portion is connected through belts respectively to the L-shaped end of L-shaped fixation section and to the distal fixation part such that the treatment device installing portion can make parallel movement.

This invention will still further provide a joint brace of which the joint angle fixation portion comprises a proximal fixation part to be attached to the upper limb close to a joint and a distal fixation part to be attached to the lower limb close to the joint, and an angle fixation means to fix the fixation angle between the proximal and distal fixation parts, thereby enabling the fixation angle to be fixed at a specified angle.

Particularly, this invention will provide a joint brace which has a joint supporting portion to support a flexed joint, the joint supporting portion having a recess to receive the head of a joint for proper alignment setting, and an angle fixation means serving as a means to fix the open angle between the proximal and distal fixation parts. This invention will still further provide a joint brace, of which each of the proximal and distal fixation parts is made of a U-shaped member having a U-shaped inner surface with a specified length; an angle fixation means is made of interconnected members each having a specified length, and has a hinge at one end which allows easy adjustment of the fixation angle; and the hinge has one end connected to the proximal fixation part and the other end connected to the distal fixation part.

This invention will still further provide a joint brace characterized in that a treatment device installing portion serves as a means to alter the position and angle of a treatment device attached thereto by allowing the device to make parallel movement or rotate, the treatment device installing portion being so configured as to place the treatment device against the lateral aspect of the joint.

The therapeutic device particularly suitable for combined use with the joint brace of this invention may include a joint treatment system incorporating an ultrasonic therapeutic device.

### Brief Description of the Drawings

Fig. 1 gives a perspective view of a joint brace representing an example of this invention.
Fig. 2 illustrates how the joint brace is attached to an elbow joint.
Fig. 3 illustrates how the joint brace and a treatment device are attached to an elbow joint.
Fig. 4 gives the right lateral view of a joint brace representing an example of this invention.
Fig. 5 gives the left lateral view of the joint brace representing the example of this invention.
Fig. 6 gives the frontal view of the joint brace representing the example of this invention.
Fig. 7 gives the rear view of the joint brace representing the example of this invention.

### The symbols in the figures represent as follows:

1. Proximal fixation part
2. Distal fixation part
3. Joint supporting portion
4. Hinge
5. Connector
6. Angle fixation means
7. L-shaped fixation section
10. Belt
11. Belt
12. Belt
13. Link
14. Fastening means
15. Pivot pin
16. Angle adjustment pin
17. Joint head receiving hole
18. Rotation axis
19. Part to receive the head of a treatment device
20. Connector fixation screw
22. Ultrasonic treatment device.

### Best Mode for Carrying Out the Invention

The present invention provides a joint brace to fix a joint flexed nearly at a right angle and keep it at that position in order to allow, in the treatment of the joint using a therapeutic device based on ultrasonic, electric or magnetic radiation, the device to direct the therapeutic radiation towards the cavity of the joint. To fix a joint nearly at a right angle, the brace has fixation parts to be attached to the proximal (upper) and/or distal (lower) limbs (segments) encompassing a joint of an arm or leg, and fixes the joint at a specified angle therewith such that a treatment device installing portion is movable along an axis passing through the fixation parts and the joint angle, thereby allowing a therapeutic device attached thereto to be properly directed towards the cavity of the joint for treatment.

For a practical example where the joint brace is applied to an elbow joint, the joint angle fixation portion to fix the joint is made of a U-shaped member having a U-shaped inner surface with a specified length; a distal fixation part is attached to the lower limb of the joint, and a section extending from the distal fixation part towards the joint and having an L-shaped inner surface has its L-shaped end fixed onto the dorsal aspect of the upper arm, thereby fixing the elbow joint nearly at a right angle.

This invention further provides a joint brace having a head receiving means which, after the brace has been attached to a joint to fix it at a specified angle, receives a head part of a treatment system, and which is movable in an axial direction along the limb in continuity of the joint to allow the head to be directed towards the cavity of the joint.

Therapy towards the cavity of the joint occurs, if the joint is a knee joint, from the lower frontal aspect, and from left and right lateral aspects of the knee because the patella tends to shift upwards when the knee is fixed while it is flexed at a right angle. To meet this situation, the treatment device installing portion is positioned in such a way as to allow a treatment device therein to face the frontal aspect of the distal end of the joint, or the left or right lateral aspect of the joint. For the treatment of an elbow joint, the joint is fixed at a right angle, and the treatment device installing portion is positioned in such a way as to allow a treatment device therein to apply therapy from the left or right lateral aspect of the joint or from the distal end of the upper arm on the dorsal aspect of the cubital head. Particularly because this treatment device installing portion allows the treatment device therein to face the lateral aspect of a joint, attachment of the device to the joint is so stabilized that therapy towards the cavity of the joint is insured without being disturbed by the existence of nearby bones.

Mounting of a treatment device to the treatment device installing portion can occur by any method depending on the shape and type of the device: the device may have threads for fixation with screws; the device may be fixed simply by insertion; or the device and the receiving portion may have mating stoppers for fixation.

The limb (distal) fixation part is U-shaped to fit to the arm, and the radius of curvature of the inner surface of the U-shaped part can be varied according to the size of the arm. Usually, the radius of curvature should preferably be 25 to 65 mm, particularly 35 to 45 mm. Its crosswise width, that is, the width along the axis of the arm should preferably be 40 to 100 mm. The material composing the fixation part preferably includes an elastic material to snugly fit to the arm such as plastics that will not give any irritating effect to the skin.

To fix a joint precisely at a specified angle, the joint brace is preferably configured such that the joint angle fixation portion includes a proximal fixation part to be attached to the upper limb of the joint, and a distal fixation part to be attached to the lower limb of the joint, and a joint angle fixation means to fix the fixation angle between the proximal and distal fixation parts.

To further rigorously fix the joint at a specified position, the joint brace preferably includes a joint supporting portion to support a flexed joint and a recess to facilitate alignment of the joint head which is inserted into the joint supporting portion and rests there. The recess is not limited in size to any specific range, but preferably has a size of 10 to 100 mm, that is, is sufficiently large as to surround the flexed part of the joint and its adjacent parts.

Further provided is a treatment device installing portion having belts to receive a head of a treatment system which also serve as a connecting means to connect the treatment device with the distal fixation part and the joint supporting portion.

With this example it is possible to vary the open angle between the proximal and distal fixation parts to any angle including a right angle by adjusting the joint angle fixation means, and thus to minutely adjust the angle in question to meet the individual differences in joint position. -

This invention will be further detailed below with reference to the attached figures. In examples below, a joint brace for an elbow joint is exclusively discussed, but this invention is not limited to an elbow joint, but may be applied to any joints including the knee joint.

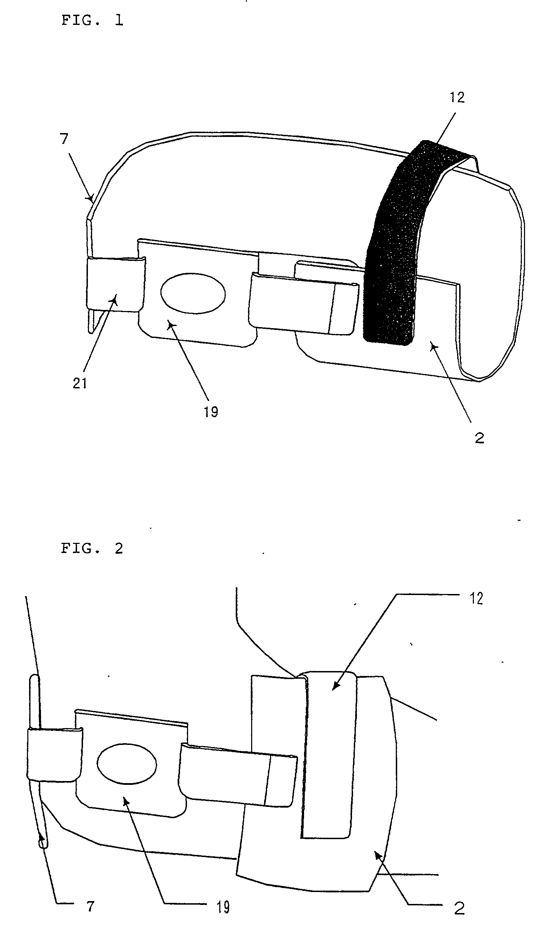

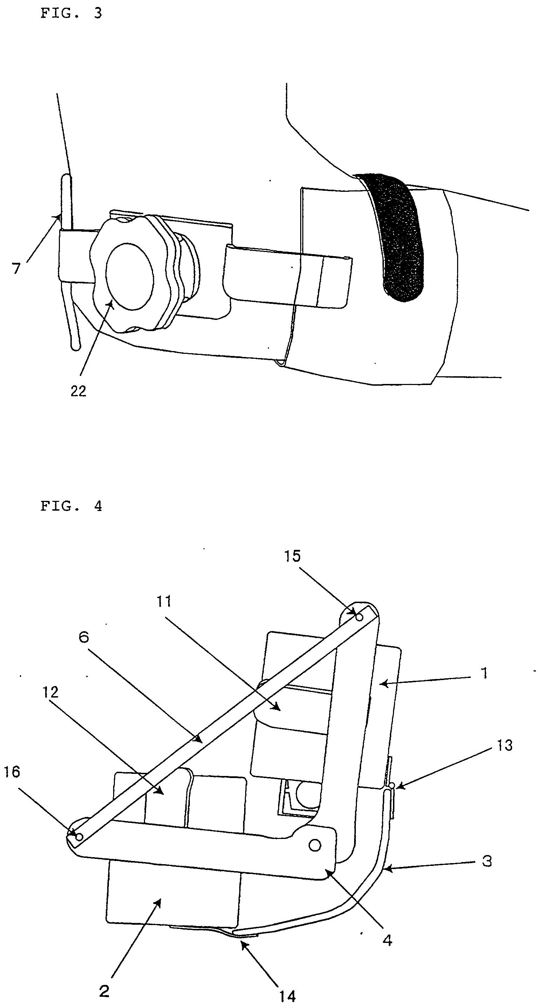

A preferred example of the joint brace of this invention is shown in Fig. 1; the manner how the brace is applied to an elbow joint is shown in Fig. 2; and the manner how treatment is applied by attaching a treatment device to the brace is shown in Fig. 3.

The joint brace comprises five parts a U-shaped distal fixation part 2 to fix the brace to the forearm, an L-shaped fixation section 7 extending from a lateral end of the distal fixation part 2, a treatment device installing portion 19, a fastening belt 10, and a fixation belt 12.

The distal fixation part 2 includes a fastening means to fasten the part to the forearm while the elbow joint is flexed at a specified angle. The L-shaped fixation section 7 extending from one end of distal fixation part 2 extends along the forearm towards the elbow joint; and the distal fixation part 2 is fixed with a fixation belt 12 such that the L-shaped part comes into contact with the dorsal aspect of the brachial part of the joint. The elbow joint flexed at a right angle is now fixed with this brace, and even if the arm were moved or the wrist were rotated, neither the position nor the angle of the elbow joint would change in association, and thus fixation of the elbow joint is achieved. In this example, the distal fixation part 2 and the L-shaped fixation section 7 are integratively united in order to cover therewith the forearm including the joint, and they are made of a thermoplastic resin. The distal fixation part 2 has a U-shaped section with the radius of curvature of 40 mm and a crosswise width, that is, the width along the axis of arm of 80 mm. The fixation belt 12 is applied to the U-shaped section of the fixation part 2, and is made of a magic tape (TM) having a width, of 30 mm for easy removal.

The treatment device fixation belt 10 has one end connected to the L-shaped end of L-shaped fixation section, and the other end to the treatment device installing portion, and serves to keep the distance from the joint to the treatment device constant. Accordingly, the belt must be adjusted in its length according to the shape of the arm and joint of the user prior to use. In this example, the belt to be attached to the treatment device installing portion 19 is made of a magic tape (TM) with a width of 40 mm for easy removal and attachment. The treatment device installing portion 19 is to receive a treatment device such as a head for ultrasonic radiation from a ultrasonic therapeutic system, and in this example a plate made of a hard resin having a window with a diameter of 40 mm is used.

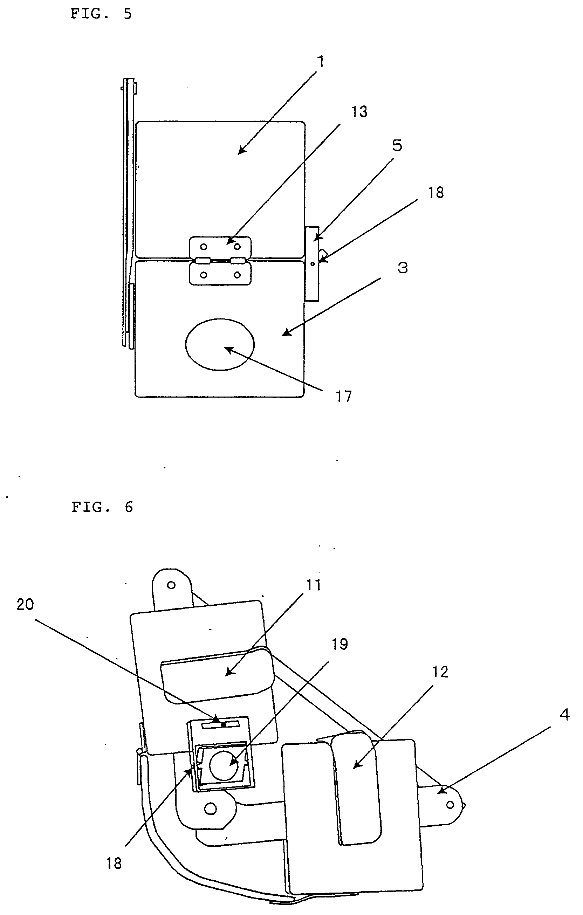

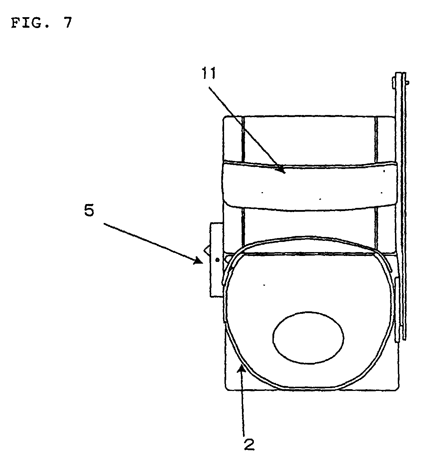

Other preferred examples of the joint brace of this invention are represented in Figs. 4 to 7. Each of the joint braces introduced here includes a joint supporting portion to support a flexed elbow or knee joint at its angle, proximal and distal fixation parts to be attached to the proximal (upper) and distal (lower) segments of an arm or leg, and an angle fixation part to adjust the open angle between the two fixation parts. Particularly, the joint supporting portion is so configured as to have a recess to fit to the joint and made of a hard member, or have an opening to receive the joint, thereby keeping the position of the brace relative to the joint constant. The joint supporting portion further includes a connector to place a head from a treatment or diagnosis system against the joint, which allows the head to make parallel movement or rotate for angle adjustment.

The above brace for an elbow joint comprises six parts: proximal fixation part 1, distal fixation part 2, joint supporting portion 3, hinge 4, connector 5 and angle fixation part 6.

The proximal and distal fixation parts are fixation means to be attached to the upper and fore-arm respectively, and they are fixed to their respective body parts through belts 11 and 12. The belts may be made of any material including a magic tape (TM) as long as it insures firm attachment of the fixation parts to the arm.

The proximal and distal fixation parts 1 and 2 are both made of a plastic product processed to have a U-shaped cross-section. It is possible to apply cloth comfortable to touch on the surface of plastic to improve the touch feeling of the latter and wear comfortableness of the brace.

The proximal and distal fixation parts 1 and 2 are connected to each other through a hinge 4. Thanks to the existence of the hinge, the user can insert his arm through the stretched brace before use, and bend the elbow until it takes a specified angle to be fixed there. The joint supporting portion 3 is attached to the proximal fixation part 1 through a link 13. The link 13 is provided, when the user wants to stretch his arm, to allow the joint supporting portion to open, as soon as the fastening means 14 is detached from the joint supporting portion, which enables easy attachment and removal of the brace to and from the arm. The linking means is not limited to the one depicted here but may be represented by a tape. The fastening means of this example consists of a magic tape (TM), but it may include any fastening means such as an adhesive tape or a hook.

The joint supporting portion 3 is made of a plastic member having a hole 17 thereon which receives the olecranon to thereby fix the elbow joint and determine its position. In this example, the joint supporting member 3 is a plastic plate having a hole thereon, but it is not limited to such a material. It is also possible to apply cloth comfortable to touch on the surface of plastic to improve the touch feeling of the latter and wear comfortableness of the brace.

The angle fixation means 6 is attached through a pivot pin 15 to the proximal side of hinge. The angle fixation means 6 can freely rotate round the pivot pin. Fixing the joint at a specified angle is achieved by fastening the end of a hinge fixation belt to an angle adjustment pin 16 located towards the forearm. In this example, to fix the joint at a right angle, the angle fixation means 6 is used together with the belt. The fixation method, however, is not limited to the use of such a means but may include any means as long as it can prevent the hinge from rotating.

In this example, the hinge 4 is composed of two aluminum limbs rotatable round a central point. It is not limited, however, to this type of hinge but may include any type of hinge as long as it has a means to bend, or allows the distance between the proximal and distal fixation parts 1 and 2 to be constant.

The connector 5 has a head mount 19 to which a head of a treatment system to radiate therapeutic medium is to be mounted. Moreover, the head mount has a rotational axis, and can rotate round this axis. Through this mechanism, it is possible to direct the head from a treatment system towards the lesion with a proper angle. Furthermore, the connector 5 is fixed with a fixation screw 20 to enable parallel movement which, when loosened, allows parallel movement of the connector 5. Through this mechanism it is possible to place properly the treatment device with respect to the lesion.

With this example, the treatment device applicable to the joint brace may include a joint treatment system based on radiation of ultrasonic waves. Such a treatment system promotes the recovery of the lesion by radiating ultrasonic waves to it, and comprises a cylindrical treatment head incorporating a transducer for radiation of ultrasonic waves, and a separate control body. The head receiving part of the joint brace of this invention is so configured as to snugly fit to the head for such radiation therapy.

The treatment device applicable to the joint brace of this invention is not limited to a treatment system based on radiation of ultrasonic waves as above, but may include heads to radiate magnetic fluxes or electromagnetic waves, as long as they are used in contact with a joint.

### Effect of Invention

According to this invention, it is possible to securely attach a treatment device to an elbow or knee. It is further possible to replace the device at the same position even after it has been removed from that position. Use of this invention is not limited to a treatment device but is widely applicable to objects which require the close apposition against an elbow or knee. Further, the joint brace may be singly used for the fixation of a joint.

## Claims

1. A joint brace comprising a joint angle fixation portion to fix a joint of a limb while the joint is bent at a right angle, by being fixed at the upper limb and/or lower one encompassing, and comprising a treatment device installing portion to facilitate the therapy based on the use of that device, and characterized in that the treatment device installing portion is so configured as to allow the device to apply treatment towards the capsule of the joint.

2. A joint brace as described in claim 1 wherein the joint angle fixation portion comprises a distal fixation part to be attached to the lower limb of a joint, and an L-shaped fixation section extending from the distal fixation part along the lateral aspect of the lower limb until it contacts with the dorsal aspect of the upper limb close to the joint.

3. A joint brace as described in claim 1 wherein the joint angle fixation portion comprises a distal fixation part to be attached to the lower limb of a joint for fixing the joint brace, an L-shaped fixation section extending from the distal fixation part along the lateral aspect of the lower limb until it contacts with the dorsal aspect of the upper limb close to the joint, and a treatment device installing portion is attached from an L-shaped end of the L-shaped fixation section and arranged in parallel with and opposite to the L-shaped fixation section.

4. A joint brace as described in claim 3 wherein the treatment device installing portion is connected through belts respectively to the L-shaped end of L-shaped fixation section and to the distal fixation part such that the treatment device installing portion can make parallel movement.

5. A joint brace as described in claim 1 wherein the joint angle fixation portion comprises a proximal fixation part to be attached to the upper limb close to a joint and a distal fixation part to be attached to the lower limb close to the joint, and an angle fixation means to fix the fixation angle between the proximal and distal fixation parts, thereby enabling the fixation angle to be fixed at a specified angle.

6. A joint brace as described in claim 5 which comprises a joint supporting portion to support a flexed joint and the joint supporting portion has a recess to receive the head of a joint for proper alignment setting.

7. A joint brace as described in claim 5 wherein the angle fixation means serves as a means to fix the open angle between the proximal and distal fixation parts.

8. A joint brace as described in claim 5 wherein each of the proximal and distal fixation parts is made of a U-shaped member having a U-shaped inner surface with a specified length, and the angle fixation means is made of interconnected members each having a specified length.

9. A joint brace as described in claim 8 comprising a mechanism which has a hinge for free adjustment of the fixation angle at one end, and the one end connected to the proximal fixation part and the other end connected to the distal fixation part.

10. A joint brace as described in claim 5 wherein the treatment device installing portion serves as a means to alter the position and angle of a treatment device attached thereto by allowing the device to make parallel movement or rotate.

11. A joint brace as described in claim 10 wherein the treatment device installing portion is placed to the lateral aspect of a joint.

12. A joint brace as described in claim 1 wherein the treatment device is an ultrasonic therapeutic device.
